# EUROPEAN PATENT SPECIFICATION

(11) **EP 4 212 020 B1**
(45) Date of publication and mention of the grant of the patent: **11.11.2026**
(21) Application number: 21867107.1
(22) Date of filing: 08.09.2021
(51) Int. Cl.: A01N 63/20, A01N 63/22, A01N 25/04, A01P 15/00, C12N 1/20, C12R 1/07

(54) **COMPOSITION FOR CONTROLLING PLANT DISEASE AND METHOD FOR PREPARING SAME**
ZUSAMMENSETZUNG ZUR BEKÄMPFUNG VON PFLANZENBEFALL UND VERFAHREN ZUR HERSTELLUNG DAVON
COMPOSITION DE LUTTE CONTRE UNE MALADIE VÉGÉTALE ET SON PROCÉDÉ DE PRÉPARATION

(30) Priority: 08.09.2020 KR 20200114907
(43) Date of publication of application: 19.07.2023
(73) Proprietor: Korea Research Institute of Bioscience and Biotechnology, Daejeon 34141 (KR)
(72) Inventor: RYU, Choong Min, Daejeon 34141 (KR); LEE, Sang Moo, Daejeon 34141 (KR)
(74) Representative: Maiwald GmbH
(86) International application number: PCT/KR2021/012187
(87) International publication number: WO 2022/055241

(56) References cited:
- WO-A1-2017/127535
- CN-A- 103 053 621
- CN-A- 105 385 643
- MEENA . ET AL: "Plant growth promoting traits shown by bacteria Brevibacterium frigrotolerans SMA23 Isolated from Aloe vera rhizosphere", vol. 37, no. 03, 3 September 2017 (2017-09-03), IN, pages 226 - 231, XP093151620, ISSN: 0253-150X, Retrieved from the Internet <URL:https://arccarticles.s3.amazonaws.com/webArticle/Final-attachment-published-D-4594.pdf> DOI: 10.18805/asd.v37i03.9223
- ZHANG CHUN ET AL: "Analysis of the complete genome sequence ofZB201705 isolated from drought- and salt-stressed rhizosphere soil of maize", ANALS OF MICROBIOLOGY, DISTAM, MILAN, IT, vol. 69, no. 13, 23 November 2019 (2019-11-23), pages 1489 - 1496, XP036969939, ISSN: 1590-4261, [retrieved on 20191123], DOI: 10.1007/S13213-019-01532-0
- BATOOL RAUFA ET AL: "Biocontrol potential of Bacillus gibsonii and Brevibacterium frigoritolerans in suppression of Fusarium stalk rot of maize: a sustainable approach", ASIAN J AGRIC BIOL, 30 September 2019 (2019-09-30), pages 320 - 333, XP093151624, Retrieved from the Internet <URL:https://www.asianjab.com/wp-content/uploads/2019/10/1.-AJAB-2019-02-071_Okay.pdf> [retrieved on 20240415]
- ZHENG XUEFANG ET AL: "Selection and Identification of Biological Control Bacillus Strains agaist Plant Bacterial Wilt Disease", ZHONGGUO-SHENGWU-FANGZHI : JIKAN = CHINESE JOURNAL OF BIOLOGICAL CONTROL, vol. 32, no. 5, 31 October 2016 (2016-10-31), CN, pages 657 - 665, XP055911291, ISSN: 1005-9261, DOI: 10.16409/j.cnki.2095-039x.2016.05.016
- KIM HYE-SOOK ET AL: "Characterization of Bacillus luciferensis strain KJ2C12 from pepper root, a biocontrol agent of Phytophthora blight of pepper", THE PLANT PATHOLOGY JOURNAL, 1 January 2009 (2009-01-01), South Korea, pages 62 - 69, XP093151670, Retrieved from the Internet <URL:https://www.ppjonline.org/upload/pdf/PPJ025-01-09.pdf> [retrieved on 20240415], DOI: 10.5423/PPJ.2009.25.1.062
- SRODOWISKA ROCZNIK OCHRONA ET AL: "Biological Control as an Alternative Method of Protecting Crops Against Fungal Pathogens", ANNUAL SET:THE ENVIRONMENT PROTECTION (ROCZNIK OCHRONA SRODOWISKA), 1 January 2019 (2019-01-01), Poland, pages 1366 - 1377, XP093151678, Retrieved from the Internet <URL:https://ros.edu.pl/images/roczniki/2019/084_ROS_V21_R2019.pdf> [retrieved on 20240415]
- NAGEL M. ET AL: "Bacillus niacini sp. nov., a Nicotinate-Metabolizing Mesophile Isolated from Soil", vol. 41, no. 1, 1 January 1991 (1991-01-01), pages 134 - 139, XP093151681, ISSN: 0020-7713, Retrieved from the Internet <URL:https://www.microbiologyresearch.org/docserver/fulltext/ijsem/41/1/ijsem-41-1-134.pdf?expires=1713181173&id=id&accname=guest&checksum=F8086EC84E4DA948999C4729C966698A> DOI: 10.1099/00207713-41-1-134
- MARKANDE ANOOP R. ET AL: "Characterization of Solibacillus silvestris strain AM1 that produces amyloid bioemulsifier", vol. 58, no. 6, 25 April 2018 (2018-04-25), BERLIN, DE, pages 523 - 531, XP093151700, ISSN: 0233-111X, Retrieved from the Internet <URL:https://api.wiley.com/onlinelibrary/tdm/v1/articles/10.1002%2Fjobm.201700685> DOI: 10.1002/jobm.201700685
- ZHENG XUEFANG, BO LIU, YUJING ZHU, CIBIN GE, GUOHONG LIU: "Selection and Identification of Biological Control Bacillus Strains agaist Plant Bacterial Wilt Disease", CHINESE JOURNAL OF BIOLOGICAL CONTROL, vol. 32, no. 5, 31 October 2016 (2016-10-31), CN , pages 657 - 665, XP055911291, ISSN: 1005-9261, DOI: 10.16409/j.cnki.2095-039x.2016.05.016
- NISHIJIMA TAKANORI, TOYOTA KOKI, MOCHIZUKI MASAMI: "Predominant Culturable Bacillus Species in Japanese Arable Soils and Their Potential as Biocontrol Agents", MICROBES AND ENVIRONMENTS, vol. 20, no. 1, 1 January 2005 (2005-01-01), pages 61 - 68, XP055911037, ISSN: 1342-6311, DOI: 10.1264/jsme2.20.61
- BATOOL RAUFA, UR REHMAN SHAFIQ, RAFIQUE MAZHAR, AMNA, ALI JAVED, MUKHTAR TEHMEENA, MAHMOOD SHEHZAD, SULTAN TARIQ, HUSSAIN MUNIS FA: "Biocontrol potential of Bacillus gibsonii and Brevibacterium frigoritolerans in suppression of Fusarium stalk rot of maize: a sustainable approach", ASIAN J AGRIC & BIOL, vol. 7, no. 3, 30 September 2019 (2019-09-30), pages 320 - 333, XP055911040
- LEE SANG-MOO; KONG HYUN GI; SONG GEUN CHEOL; RYU CHOONG-MIN: "Disruption of Firmicutes and Actinobacteria abundance in tomato rhizosphere causes the incidence of bacterial wilt disease", THE ISME JOURNAL, NATURE PUBLISHING GROUP UK, LONDON, vol. 15, no. 1, 1 January 1900 (1900-01-01), London, pages 330 - 347, XP037322771, ISSN: 1751-7362, DOI: 10.1038/s41396-020-00785-x

## Description

### [Technical Field]

The present disclosure relates to a composition for controlling plant disease and a method for controlling plant disease.

### [Background Art]

Bacterial wilt is a disease with rapid wilting (withering) symptoms and is caused by the plant pathogenic bacterium *Ralstonia solanacearum.* Bacterial wilt is a globally distributed soil disease that infects more than 200 plant species, including major solanaceous crops such as tomatoes, potatoes, peppers, eggplants, and tobacco, to thereby cause serious economic losses in agricultural and horticultural production. At first, leaves near growing points wilt, and recover for several days at night and on rainy days, and then the whole leaves wilt, so the entire plant cannot recover and be dried. It may be seen that, when a stem is cut or an epidermis is cut, a vascular tube is browned, and when the stem is cut and floated in water, white bacterial liquid (ooze) flows out. Bacterial wilt occurs more severe under a high-temperature and high-humidity environment with an increased incidence and appears frequently in facility cultivation such as greenhouse. Once bacterial wilt occurs, it is highly contagious by contact, so all diseased plants should be removed and the temperature and humidity should be lowered. However, it is very difficult to put this into practice in farms.

In order to prevent the occurrence of bacterial wilt, as a bacterial wilt control method a cultural method such as crop rotation, intercrop cropping, cultivation and packaging hygiene and a method of cultivating resistant varieties have been mainly applied. It is known that the use of copper (coperhydroxide) and agricultural antibiotics (streptomycin, oxytetracycline, validamycin, etc.) is somewhat effective, but other chemical control measures are very limited. Recently, biopesticides that are effective in controlling bacterial wilt have been developed and patented or released as products and are being supplied to farmers. Most of the biopesticides use microorganisms such as bacteria genus *Pseudomonas, Burkholderia, Bacillus, Ricinibacillus, Pennybacillus,* Brevibacillus, and Trichoderma. As the biopesticides, products such as probiotic charcoal (Farm Hannong Co., Ltd.), Heukhyang (BIG. Co., Ltd.), Cheongotan (BIG. Co., Ltd.), Pangi Coma (Nambo Co., Ltd.), and KomaH (Nambo Co., Ltd.) are being released and sold in the market in Korea. Microbial bio-pesticides are considered environmentally friendly control measures compared to synthetic pesticides having a wide control spectrum because the microbial bio-pesticides do not affect other than target organisms, but cannot cope with all species present in the population of target pathogens in actual packaging.

Rhizosphere microbiota plays an important role in rigidity, growth, and immunity of plants. The positive and negative effects of single crop cultivation systems on soil microbes have been studied for a long time. Generally, a phenomenon known as negative plant-soil feedback changes the soil environment to a disease-prone condition. On the other hand, after persistent and serious occurrence of the disease due to continuous single crop cultivation, the progress of the soil disease may be suppressed. In such soil, despite the presence of the disease, plant hosts simultaneously exhibit resistance to the disease and the occurrence of the disease may be minimized. Disease-suppressive soils are mainly known to be effective against pathogens such as *Rhizoctonia solani* (rice sheath blight), *Pythium ultimum* (damp-off disease), *Gaeumannomyces graminis* var *tritici* (take-all), *Plasmodiophora brassicae* (root gall disease), and *Fusarium oxysporum* (fusarium wilt disease) which are soil-derived fungal diseases, and *Ralstonia solanacearum* (bacterial wilt) which is a bacterial disease.

Control of plant diseases is mainly undertaken with chemical pesticides, but concerns about the use thereof are increasing for reasons such as acquisition of resistance against drugs by pathogenic microorganisms, residual toxicity of chemical pesticides, toxicity to humans and animals, and destruction of ecosystems. Accordingly, the development and use of microbial agents as a substitute for the chemical pesticides are increasing.

As a patent for a previously registered microbial agent, Korean Patent Publication No. 10-1188641 discloses that a microbial fertilizer including *Bacillus subtilis* (GDYA-1) or a culture solution or mixture thereof as an active ingredient has antibacterial activity against plant pathogens such as white leaf blight, blast disease, kidney disease, rice kernel blight, sesame seed blotch, and sheath blight, and Korean Patent Publication No. 10-2126582 discloses that a microbial fertilizer simultaneously controls *Isaria javanica* FT333 strains (*Isaria javanica* FT333) or thrips and plant anthracnose using the same.

It has been reported that compositions of rhizosphere microorganisms in soils that suppresses and causes disease in plants differ, and the imbalance (dysbiosis) of microorganisms living in rhizosphere of soils makes a difference in the incidence of disease. Studies on the microflora of *Arabidopsis thaliana* leaves have reported that an increase in Proteobacteria, an antagonistic microorganism, decreases Firmicute of leaf, resulting in a microbial imbalance. This study provides a clue that the addition of specific beneficial bacteria alone or a mixture thereof may reverse microbial imbalance even in the soil, which has been unbalanced due to the prevalence of pathogens, and induce plants to grow healthily.

A study by Zheng Xuefang et al. (2016) Chinese journal of biological control, 32(5, 31) : 657-665 reports on the selection and identification of biological control *bacillus* strains against plant bacterial wilt disease.

Another study by Kim Hye-Sook et al. (2009) The Plant Pathology Journal 25(1) : 62-69 characterizes the *Bacillus luciferensis* strain KJ2C12 from pepper root in relation with its biocontrol activity against *Phytophthora capsici* on pepper.

A further study by Srodowiska Rocznik Ochrona et al. (2019) Rocznik Ochrona Srodowiska (Annual Set the Environment Protection), 21 : 1366-1377 reports on biological control as an alternative method of protecting crops against fungal pathogens.

### [Disclosure]

### [Summary]

The invention is defined by the appended claims.

### [Technical Problem]

The present disclosure provides a composition for controlling plant disease comprising a mixture of strains comprising *Brevibacterium frigoritolerans* HRS1 (KCTC 14301BP), *Bacillus niacini* HRS2 (KCTC 14302BP), and *Bacillus luciferensis* (HRS4, KCTC 14304BP) strains capable of eliciting immunity(resistance) of plants against plant pathogens as an active ingredient. Optionally, the mixture of strains comprised in the composition according to the present disclosure may further comprise *Solibacillus silvestris* HRS3 (KCTC 14303BP). *Brevibacterium frigoritolerans* HRS 1 was deposited on September 08, 2020 under the number KCTC 14301 BP at the Biological Resource Center of the Korea Research Institute of Bioscience and Biotechnology. *Bacillus niacini* HRS 2 was deposited on September 08, 2020 under the number KCTC 14302BP at the Biological Resource Center of the Korea Research Institute of Bioscience and Biotechnology. *Bacillus luciferens* HRS 4 was deposited on September 08, 2020 under the number KCTC 14304BP at the Biological Resource Center of the Korea Research Institute of Bioscience and Biotechnology. *Solibacillus silvestris* HRS 3 was deposited on September 08, 2020 under the number KCTC 14303BP at the Biological Resource Center of the Korea Research Institute of Bioscience and Biotechnology.

The present disclosure provides a method for controlling plant disease including applying a composition to a plant or around the plant and optionally, in which, in the applying, the composition may be applied to soil, roots, seeds, sprayed onto plants, or may be applied by a combination thereof.

In one embodiment outside the scope of the claimed invention, the present disclosure provides a method for preparing a composition for controlling plant diseases including cultivating one or more of *Brevibacterium frigoritolerans* HRS1 (KCTC 14301BP), *Bacillus niacini* HRS2 (KCTC 14302BP), *Solibacillus silvestris* HRS3 (KCTC 14303BP) and *Bacillus luciferensis* (HRS4, KCTC 14304BP) strains capable of improving immunity (resistance) of plants against plant pathogens.

The present disclosure provides a use of a mixture of strains comprising *Brevibacterium frigoritolerans* HRS1 (KCTC 14301BP), *Bacillus niacini* HRS2 (KCTC 14302BP), and *Bacillus luciferensis* HRS4 (KCTC 14304BP) strains for preparing a composition for controlling plant disease. Optionally, the mixture of strains for the use according to the present disclosure may further comprise *Solibacillus silvestris* HRS3 (KCTC 14303BP).

### [Technical Solution]

In one embodiment outside the scope of the claimed invention, the present disclosure provides *Brevibacterium frigoritolerans* HRS1 (KCTC 14301BP), *Bacillus niacini* HRS2 (KCTC 14302BP), *Solibacillus silvestris* HRS3 (KCTC 14303BP), or *Bacillus luciferensis* HRS4 (KCTC 14304BP) strains capable of improving resistance of plants against plant pathogens.

In one embodiment of the present disclosure, the plant pathogen may be one or more of *Ralstonia solanacearum* and *Xanthomonas axonopodis* pv. *vesicatoria* (Xav).

Another aspect of the present disclosure provides a composition for controlling plant disease comprising a mixture of strains comprising *Brevibacterium frigoritolerans* HRS1 (KCTC 14301BP), *Bacillus niacini* HRS2 (KCTC 14302BP), and *Bacillus luciferensis* HRS4 (KCTC 14304BP) strains as an active ingredient. Optionally, the mixture of strains comprised in the composition according to the present disclosure may further comprise *Solibacillus silvestris* HRS3 (KCTC 14303BP).

In one embodiment of the present disclosure, the plant disease may be any one of bacterial wilt on root and spot diseases on leaf.

Another aspect of the present disclosure provides a method for controlling plant disease including applying the composition to a plant or around the plant.

In one embodiment of the present disclosure, in the applying, the composition may be applied to soil, roots, and, seeds, sprayed onto plants, or may be applied by a combination thereof.

### [Advantageous Effects]

According to the present disclosure, the microbial preparation for plant disease control including *Brevibacterium frigoritolerans* HRS1 (KCTC 14301BP), *Bacillus niacini* HRS2 (KCTC 14302BP), *Solibacillus silvestris* HRS3 (KCTC 14303BP), and *Bacillus luciferensis* HRS4 (KCTC 14304BP) strains may show excellent effects on bacterial wilt control on tomato and aphid control on pepper through strain combination.

### [Brief Description of Drawings]

FIG. 1 is a diagram illustrating microorganisms in soil collected as a result of 16S rRNA analysis at a phylum level. Healthy rhizosphere soil (HRS), diseased rhizosphere soil (DRS).
FIG. 2 is a diagram illustrating a percentage of Gram-positive bacteria in the healthy rhizosphere soil (HRS) and diseased rhizosphere soil (DRS) through a 3% KOH string test and a selective medium.
FIG. 3 is a diagram illustrating the occurrence amount of tomato bacterial wilt in the healthy rhizosphere soil (HRS) and diseased rhizosphere soil (DRS). It shows that treatment with vancomycin, an antibiotic that suppresses Gram-positive bacteria, increases the occurrence of tomato bacterial wilt of the HRS.
FIG. 4 is a diagram illustrating resistance against disease after treating bacterial wilt on soil in which tomato roots in which *Brevibacterium frigoritolerans* HRS1, *Bacillus niacini* HRS2, *Solibacillus silvestris* HRS3, and *Bacillus luciferensis* HRS4 strains, respectively, are treated are planted.
FIG. 5 is a diagram illustrating induced systemic resistance (plant systemic immunity) response of tomatoes after tomato stem inoculation *of Ralstonia solanacearum* following drench-inoculation of tomato roots with *Brevibacterium frigoritolerans* HRS1, *Bacillus niacini* HRS2), *Solibacillus silvestris* HRS3, and *Bacillus luciferensis* HRS4 strains, respectively.
FIG. 6 is a diagram illustrating results of the induced systemic resistance response by mixing *Brevibacterium frigoritolerans* HRS1, *Bacillus niacini* HRS2, *Solibacillus silvestris* HRS3, and *Bacillus luciferensis* HRS4 strains to produce synthetic microbial community (Syncom) and then treating the synthetic microbial community (Syncom) on tomatoes.
FIG. 7 is a diagram illustrating an expression of tomato genes related to induced resistance by synthetic microbial community (Syncom) treatment.
FIG. 8 is a diagram illustrating an increase in yield of red pepper fruits by single microorganism and synthetic microorganism community (Syncom) treatments.
FIG. 9 is a diagram illustrating resistance to *Xanthomonas axonopodis* pv. *vesicatoria* (Xav), which causes bacterial spot disease of red pepper leaf with induced resistance caused by the synthetic microbial community (Syncom) treatment.
FIG. 10 is a diagram illustrating the elicitation of systemic resistance against aphids on pepper by the synthetic microbial community (Syncom) treatment.

### [Best Mode]

In one embodiment outside the scope of the claimed invention, the present disclosure provides *Brevibacterium frigoritolerans* HRS1 (KCTC 14301BP), *Bacillus niacini* HRS2 (KCTC 14302BP), *Solibacillus silvestris* HRS3 (KCTC 14303BP), or *Bacillus luciferensis* HRS4 (KCTC 14304BP) strains capable of improving resistance of plants against plant pathogens.

In one embodiment of the present disclosure, the plant pathogen may be one or more of *Ralstonia solanacearum* and *Xanthomonas axonopodis* pv. *vesicatoria* (Xav). The plant pathogens are bacteria that cause bacterial wilt on tomato root system and spot diseases on pepper leaves. According to the present disclosure, *Brevibacterium frigoritolerans* HRS1 (KCTC 14301BP), *Bacillus niacini* HRS2 (KCTC 14302BP), *Solibacillus silvestris* HRS3 (KCTC 14303BP), or *Bacillus luciferensis* HRS4 (KCTC 14304BP) strains may, in combination, improve resistance to one or more plant pathogens of *Ralstonia solanacearum* and *Xanthomonas axonopodis* pv. *vesicatoria* (Xav).

Another aspect of the present disclosure provides a composition for controlling plant diseases including Brevibacterium frigoritolerans HRS1 (KCTC 14301BP), Bacillus niacini HRS2 (KCTC 14302BP), and Bacillus luciferensis HRS4 (KCTC 14304BP) strains as an active ingredient.

The non-claimed culture solution of the strains may include both a culture solution including the strains and a culture solution obtained by filtering the strains broth culture. In addition in an embodiment outside the scope of the claimed invention, the composition may include a carrier other than the strains themselves, the culture solution of the strains, and the extract of the culture solution, and as the preferred carrier, water, white carbon, kaolin, dextrin, and the like may be used.

The non-claimed extract of the culture solution may be preferably an extract obtained by extracting a culture solution of strains with an appropriate extraction solvent, for example, ethyl acetate, butanol, water, or a mixture thereof, and more preferably an ethyl acetate layer or a butanol layer remaining after fractionating a culture supernatant with the equal amounts of ethyl acetate or butanol, or an aqueous solution layer remaining after fractionating the culture supernatant with the equal amounts of ethyl acetate and butanol.

In an embodiment outside the scope of the claimed invention, after mixing the spores or vegetative cells of the strains, the culture solution of the strains, or the extract of the culture solution with the carrier, it may be formulated into powder, pellets, granules, or solutions and used.

In one embodiment of the present disclosure, the plant disease may be any one of bacterial wilt and spot disease, and more specifically, any one of tomato bacterial wilt and pepper spot disease. Most specifically, the plant disease may be any one of tomato bacterial wilt caused by *Ralstonia solanacearum* and pepper spot disease caused by *Xanthomonas axonopodis* pv. *vesicatoria* (Xav).

Another aspect of the present disclosure provides a method for controlling plant disease including applying the composition to a plant or around the plant.

As described above, the plant disease may be any one of bacterial wilt and spot disease, and more specifically, any one of tomato bacterial wilt and pepper spot disease. Most specifically, the plant disease may be any one of tomato bacterial wilt caused by *Ralstonia solanacearum* and pepper spot disease caused by *Xanthomonas axonopodis* pv. *vesicatoria* (Xav).

In one embodiment of the present disclosure, in the applying, the composition may be applied to soil, roots, seeds, sprayed onto plants, or applied by a combination thereof.

In one embodiment outside the scope of the claimed invention, the present disclosure provides a method for preparing a composition for controlling plant disease including cultivating one or more of *Brevibacterium frigoritolerans* HRS1, KCTC 14301BP), *Bacillus niacini* (HRS2, KCTC 14302BP), *Solibacillus silvestris* (HRS3, KCTC 14303BP), or *Bacillus luciferensis* (HRS4, KCTC 14304BP) strains capable of improving resistance of plants against plant pathogens.

Another aspect of the present disclosure provides a use of a mixture of strains comprising *Brevibacterium frigoritolerans* (HRS1, KCTC 14301BP), *Bacillus niacini* (HRS2, KCTC 14302BP), and *Bacillus luciferensis* (HRS4, KCTC 14304BP) strains for preparing a composition for controlling plant disease.

### [Mode for Disclosure]

Hereinafter, one or more specific examples will be described in more detail through examples. However, these examples are intended to illustrate one or more specific examples, and the scope of the present disclosure is not limited to these examples.

### Example 1: Comparison of microbial community of healthy soil without tomato bacterial wilt and soil with tomato bacterial wilt

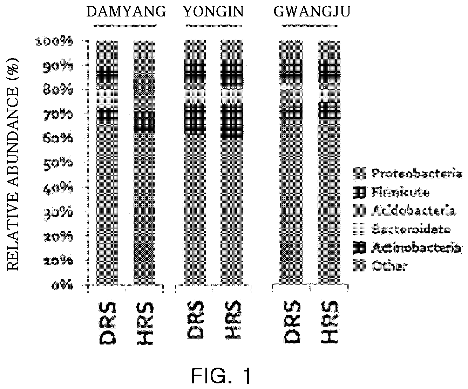

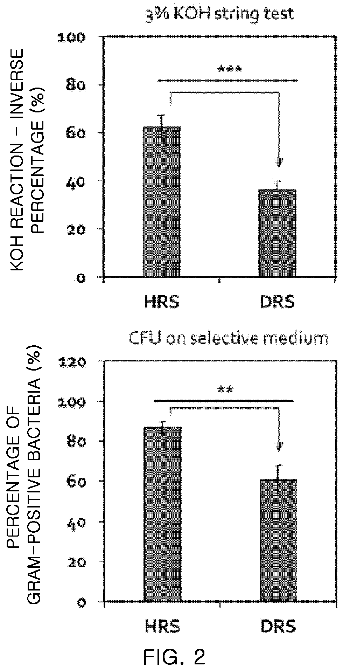

Tomato bacterial wilt occurred locally (within 1m diameter) in tomato greenhouses located in Yongin, Gwangju, and Damyang, and the soil was collected and subjected to 16S rDNA sequence analysis to analyze microbial community of soil (healthy rhizosphere soil (HRS)) in which healthy tomatoes grow and soil (diseased rhizosphere soil (DRS)) in which tomatoes diseased with tomato bacterial wilt grow. Five phyla of Firmicutes, Actinobacteria, Acidobacteria, and Bacteroidetes, were found to constitute the major microbial phase (FIG. 1). Among them, the genes of microorganisms belonging to Firmicutes and Actinobacteria were found to be much more abundant in the soil (HRS) in which healthy tomatoes grow. To prove Firmicutes and Actinobacteria, they were cultured in selective medium including 20 µg/mL polymyxin B or 5 µg/mL vancomycin and 3% KOH string test was executed. The results of the two experiments showed that Firmicutes and Actinobacteria were present in the HRS in an amount of 26.2% and 26.3%, respectively (FIG. 2).

### Example 2: Effect of Gram-positive bacteria on occurrence of tomato bacterial wilt in healthy soil where tomato bacterial wilt does not occur

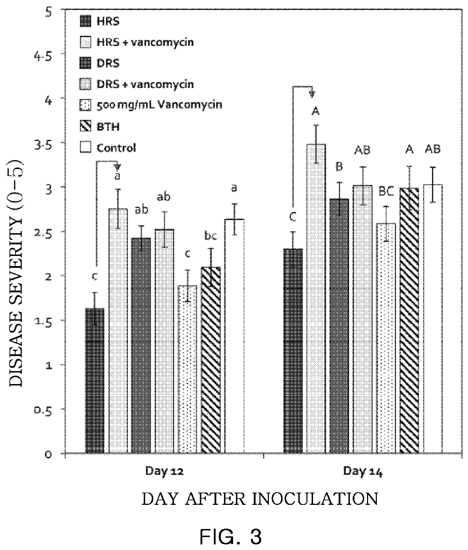

In order to examine the effect of Firmicutes and Actinobacteria confirmed in Example 1 on the occurrence of tomato bacterial wilt, symptoms of the tomato bacterial wilt were observed after suppressing Gram-positive bacteria in healthy soil. To this end, the incidence of the tomato bacterial wilt was observed in healthy soil and healthy soil treated with vancomycin which is an antibiotic specific to killing Gram-positive bacteria. It was found that, in soil where the Gram-positive bacteria were killed due to antibiotics, and thus, an imbalance in soil microorganisms has occurred, the symptoms of the tomato bacterial wilt increased 1.5 to 1.8 times as observed in FIG. 3.

### Example 3: Isolation and identification of Firmicutes and Actinobacteria in healthy soil without tomato bacterial wilt

To isolate bacteria that form endospores, MES buffer extracts of healthy soil without tomato bacterial wilt and soil with tomato bacterial wilt were cultivated at 80°C for 30 minutes, spread on TSA medium, and then cultivated at 30°C for 48 hours. Among them, four types of Gram-positive bacteria sensitive to 500 µg/mL vancomycin were selected to isolate. 16S rRNA nucleotide sequence was analyzed using 27F/1492R primers, and comparative analysis using BLASTn found that these strains were *Brevibacterium frigoritolerans* HRS1, *Bacillus niacini* HRS2, *Solibacillus silvestris* HRS3, and *Bacillus luciferensis* HRS4.

**[Table 1]**

| **Primer name** | **Primer sequence (5'->3')^{†}** |
|---|---|
| 27F | AGAGTTTGATCCTGGCTCAG |
| 1492R | GGTTACCTTGTTACGACTT |

The strains identified above were deposited on September 8, 2020 at the Korean Collection for Type Cultures (KCTC) of the Korea Research Institute of Bioscience and Biotechnology. Specifically, *Brevibacterium frigoritolerans* HRS1 was deposited as KCTC 14301BP, *Bacillus niacini* HRS2 was deposited as KCTC 14302BP, *Solibacillus silvestris* HRS3 was deposited as KCTC 14303BP, and *Bacillus luciferensis* HRS4 was deposited as KCTC 14304BP.

### Example 4: Tomato bacterial wilt suppression effect of Brevibacterium frigoritolerans HRS1, Bacillus niacini HRS2, Solibacillus silvestris HRS3, and Bacillus luciferensis HRS4 single treatment group

In order to examine the effect of 4 strains selected in Preparation Example 3 on induction of systemic resistance of plants, each of the 4 strains was treated on roots. At 5 days after root inoculation, *Ralstonia solanacearum,* bacterial disease, which is a tomato bacterial wilt-induced strain, stem-inoculated on tomato, by using a control in which roots are treated with same volume of sterile distilled water and BTH (benzo[1,2,3]thiadiazole-7-carbothioic cid S-methyl ester), a chemical that causes systemic resistance in plants, as a positive control, the incidence of disease was investigated.

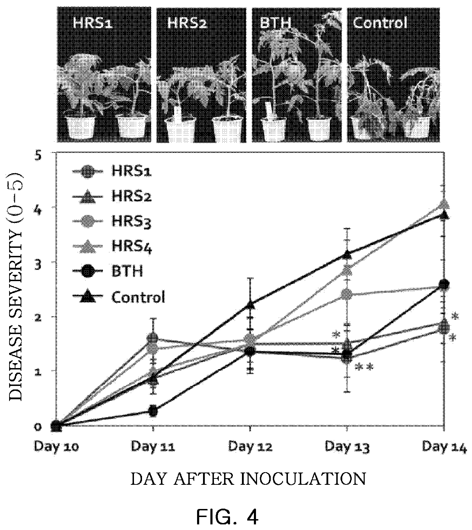

In the single treatment group, *Brevibacterium frigoritolerans* HRS1 and *Bacillus niacini* HRS2 showed resistance to bacterial wilt, as illustrated in FIG. 4 below.

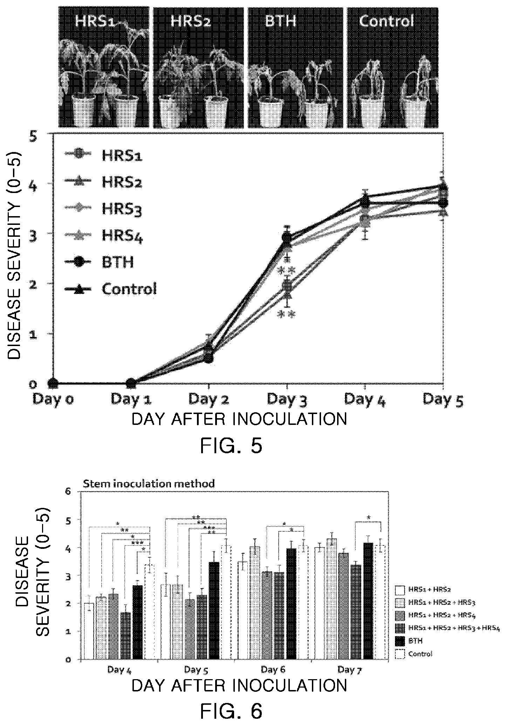

In order to find out whether 4 strains selected in Preparation Example 3 cause induced resistance of tomatoes, 50 µL of tomato bacterial wilt strains were inoculated into stems after 7 days of treatment of each strain on roots to evaluate the induced resistance. As illustrated in FIG. 5 in the *Brevibacterium frigoritolerans* HRS1 and *Bacillus niacini* HRS2 treatment groups, the induced resistance was shown after 3 days of treatment of the tomato bacterial wilt. However, it was confirmed that the induced resistance by the treatment of each strain disappears on 4th and 5th days after treating the tomato bacterial wilt.

To find out the effect of synthetic microbial community (Syncom) on the resistance of the tomato bacterial wilt rather than the single treatment group, *Brevibacterium frigoritolerans* HRS1, *Bacillus niacini* HRS2, *Solibacillus silvestris* HRS3, and Bacillus luciferensis HRS4 were mixed to treat a combination of two strains HRS1+HRS2, a combination of three strains HRS1+HRS2+HRS3 and HRS1+HRS2+HRS4, and a combination of four strains HRS1+HRS2 +HRS3+HRS4 on the soil. Treatment of two strains increased the resistance of the tomato bacterial wilt from 3 days to 5 days when each strain was treated. When all four strains were mixed, the resistance was maintained for a long time, showing that the resistance was maintained up to 7 days, the end point of the experiment (FIG. 6).

### Example 5: Expression of induced resistance-related genes by synthetic microbial community (Syncom) treatment

In order to elucidate the mechanism of expression of the induced resistance by the synthetic microbial community (Syncom) treatment, the expression of plant defense-related genes was investigated.

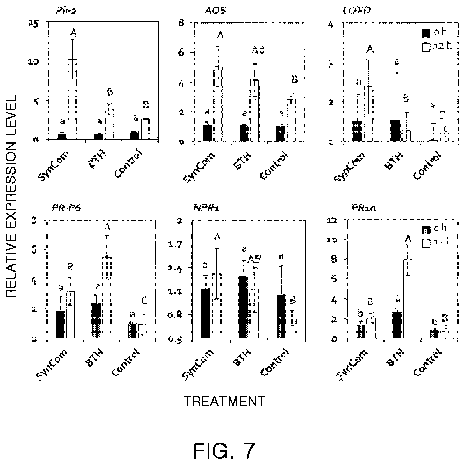

As can be seen in FIG. 7 below, it was confirmed that, after 12 days of the treatment of the tomato bacterial wilt, Jasmonic acid signaling-related genes *Pin2,* AOS, and *LoxD,* and salicylic acid signaling-related genes *PR-P6, NPR1,* and *PR1a* increased, and thus, the synthetic microbial community (Syncom) treatment caused induced resistance dependent on the resistance signal pathway to jasmonic acid and salicylic acid, causing tomatoes to develop the resistance to the tomato bacterial wilt.

### Example 6: Increased pepper yield by single or synthetic microbial community (Syncom) treatment

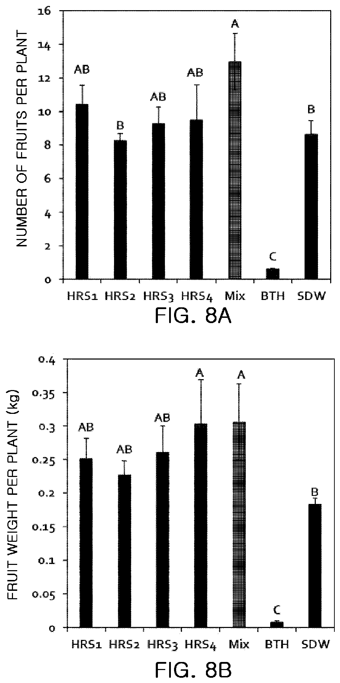

It was confirmed that the yield of red pepper increased or decreased by the single or synthetic microbial community (Syncom) treatment in the outdoor red pepper field. In the synthetic microbial phase, and it was confirmed that the number of peppers hanging per unit pepper seedling increased, and the weight per pepper drop also increased compared to the untreated group (FIG. 8).

### Example 7: Suppression of Xanthomonas axonopodis pv. vesicatoria (Xav) causing bacterial spot disease of pepper leaves through single or synthetic microbial community (Syncom) treatment

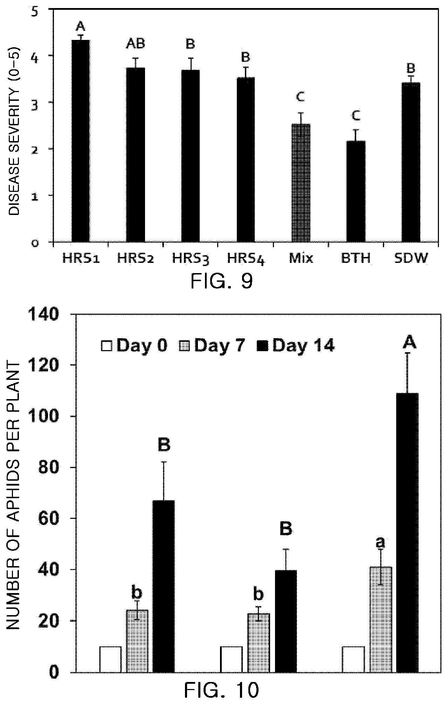

As a result of treating *Xanthomonas axonopodis* pv. *vesicatoria* (Xav), which causes the bacterial spot disease in peppers by the single or synthetic microbial community (Syncom) treatment in an outdoor red pepper field, it was confirmed that the resistance against disease in the synthetic microbial phase increased (FIG. 9).

### Example 9: Aphid control of pepper leaf in the greenhouse through synthetic microbial community (Syncom) soil treatment

As can be seen in FIG. 10 below, it was confirmed that the soil treatment of the synthetic microbial phase increased the aphid control effect of pepper in the greenhouse.

Hereinabove, the present disclosure has been described with reference to exemplary embodiments. It will be understood by those skilled in the art to which the present disclosure pertains that the present disclosure may be implemented in a modified form without departing from essential characteristics of the present disclosure. Therefore, exemplary embodiments disclosed herein should be considered in an illustrative aspect rather than a restrictive aspect. The scope of the present disclosure is defined by the claims rather than the above-mentioned description.

## Claims

1. A composition for controlling plant disease, preferably bacterial wilt or spot disease, comprising a mixture of strains comprising *Brevibacterium frigoritolerans* HRS1 (KCTC 14301 BP), *Bacillus niacini* HRS2 (KCTC 14302BP), and *Bacillus luciferensis* HRS4 (KCTC 14304BP) strains as an active ingredient.

2. The composition of claim 1, wherein the mixture of strains further comprises *Solibacillus silvestris* HRS3 (KCTC 14303BP).

3. A method for controlling plant disease including applying the composition of claim 1 or claim 2 to a plant or around the plant.

4. The method of claim 3, wherein, in the applying, the composition is applied to soil, roots, seeds, sprayed onto plants, or applied by a combination thereof.

5. A use of a mixture of strains comprising *Brevibacterium frigoritolerans* (HRS1, KCTC 14301 BP), *Bacillus niacini* (HRS2, KCTC 14302BP), and *Bacillus luciferensis* (HRS4, KCTC 14304BP) strains for preparing a composition for controlling plant disease.

6. The use of claim 5, wherein the mixture of strains further comprises *Solibacillus silvestris* HRS3 (KCTC 14303BP).

## Patentansprüche

1. Zusammensetzung zur Bekämpfung einer Pflanzenkrankheit, vorzugsweise von bakterieller Welke oder Fleckenkrankheit, umfassend ein Stammgemisch, das Stämme von *Brevibacterium frigoritolerans* HRS1 (KCTC 14301BP), *Bacillus niacini* HRS2 (KCTC 14302BP) und *Bacillus luciferensis* HRS4 (KCTC 14304BP) als Wirkstoff umfasst.

2. Zusammensetzung nach Anspruch 1, wobei das Stammgemisch ferner *Solibacillus silvestris* HRS3 (KCTC 14303BP) umfasst.

3. Verfahren zur Bekämpfung einer Pflanzenkrankheit, umfassend das Aufbringen der Zusammensetzung nach Anspruch 1 oder Anspruch 2 auf eine Pflanze oder in der Umgebung der Pflanze.

4. Verfahren nach Anspruch 3, wobei beim Aufbringen die Zusammensetzung auf Boden, Wurzeln oder Samen aufgebracht, auf Pflanzen gesprüht oder durch eine Kombination davon aufgebracht wird.

5. Verwendung eines Stammgemischs, das Stämme von *Brevibacterium frigoritolerans* (HRS1, KCTC 14301BP), *Bacillus niacini* (HRS2, KCTC 14302BP) und *Bacillus luciferensis* (HRS4, KCTC 14304BP) umfasst, zur Herstellung einer Zusammensetzung zur Bekämpfung einer Pflanzenkrankheit.

6. Verwendung nach Anspruch 5, wobei das Stammgemisch ferner *Solibacillus silvestris* HRS3 (KCTC 14303BP) umfasst.

## Revendications

1. Composition destinée à lutter contre une maladie de plante, de préférence un flétrissement bactérien ou une maladie par taches, comprenant un mélange de souches comprenant des souches de *Brevibacterium frigoritolerans* HRS1 (KCTC 14301BP), ***Bacillus** niacini* HRS2 (KCTC 14302BP) et *Bacillus luciferensis* HRS4 (KCTC 14304BP) en tant qu'ingrédient actif.

2. Composition selon la revendication 1, dans laquelle le mélange de souches comprend en outre *Solibacillus Silvestris* HRS3 (KCTC 14303BP).

3. Procédé de lutte contre une maladie de plante incluant l'application de la composition selon la revendication 1 ou la revendication 2 sur une plante ou autour de la plante.

4. Procédé selon la revendication 3, dans lequel, lors de l'application, la composition est appliquée sur le sol, les racines, les graines, pulvérisée sur des plantes ou appliquée par une de leurs combinaisons.

5. Utilisation d'un mélange de souches comprenant des souches de *Brevibacterium frigoritolerans* (HRS1, KCTC 14301BP), *Bacillus niacini* (HRS2, KCTC 14302BP) et *Bacillus luciferensis* (HRS4, KCTC 14304BP) pour préparer une composition destinée à lutter contre une maladie de plante.

6. Utilisation selon la revendication 5, dans laquelle le mélange de souches comprend en outre *Solibacillus Silvestris* HRS3 (KCTC 14303BP).
